# EUROPEAN PATENT SPECIFICATION

(11) **EP 1 626 090 B1**
(45) Date of publication and mention of the grant of the patent: **20.04.2011**
(21) Application number: 04711732.0
(22) Date of filing: 17.02.2004
(51) Int. Cl.: C12N 15/861, A61K 48/00

(54) **NOVEL VIRUS VECTOR**
NEUARTIGER VIRUSVEKTOR
NOUVEAU VECTEUR DE VIRUS

(30) Priority: 17.02.2003 JP 2003038780
(43) Date of publication of application: 15.02.2006
(73) Proprietor: FUSO PHARMACEUTICAL INDUSTRIES LTD., Osaka-shi, Osaka 541-0045 (JP); Mayumi, Tadanori, Kobe-shi, Hyogo 651-2273 (JP); Nakagawa, Shinsaku, Yao-shi, Osaka 581-0045 (JP); Tsutsumi, Yasuo, Toyono-gun, Osaka 563-0105 (JP); Kawasaki, Koichi, Kobe-shi, Hyogo 654-0162 (JP); Maeda, Mitsuko, Akashi-shi, Hyogo 673-0886 (JP)
(72) Inventor: MAYUMI, Tadanori, Nishi-ku, Kobe-shi, Hyogo 651-2273 (JP); NAKAGAWA, Shinsaku, 581-0045 (JP); TSUTSUMI, Yasuo, Toyono-gun, Osaka 563-0105 (JP); KAWASAKI, Koichi, 654-0162 (JP); MAEDA, Mitsuko, Akashi-shi, Hyogo 673-0886 (JP); HAYAKAWA, Takao, 154-0016 (JP); MIZUGUCHI, Hiroyuki, Mino-shi, Osaka 562-0011 (JP)
(74) Representative: Vossius & Partner
(86) International application number: PCT/JP2004/001739
(87) International publication number: WO 2004/072289

(56) References cited:
- WO-A-00/74722
- WO-A-01/92549
- WO-A1-00/11202
- WO-A1-98/44143
- WO-A2-01/12235
- ROMANCZUK H ET AL: "Modification of an adenoviral vector with biologically selected peptides: a novel strategy for gene delivery to cells of choice" HUMAN GENE THERAPY, MARY ANN LIEBERT, NEW YORK ,NY, US, vol. 10, no. 16, 1 November 1999 (1999-11-01), pages 2615-2626, XP002363033 ISSN: 1043-0342
- O'RIORDAN C R ET AL: "PEGYLATION OF ADENOVIRUS WITH RETENTION OF INFECTIVITY AND PROTECTION FROM NEUTRALIZING ANTIBODY IN VITRO AND IN VIVO" HUMAN GENE THERAPY, MARY ANN LIEBERT, NEW YORK ,NY, US, vol. 10, no. 8, 20 May 1999 (1999-05-20), pages 1349-1358, XP000857510 ISSN: 1043-0342
- DASH ET AL: "Decreased binding to proteins and cells of polymeric gene delivery vector surface modified with a multivalent hydrophilic polymer and retargeting through attachment of transferrin" JOURNAL OF BIOLOGICAL CHEMISTRY, AMERICAN SOCIETY OF BIOLOCHEMICAL BIOLOGISTS, BIRMINGHAM,, US, vol. 275, no. 6, 2000, pages 3793-3802, XP002158846 ISSN: 0021-9258
- VIGNE E, ET AL: 'RGD inclusion in the hexon monomer provides adenovirus type 5-based vectors with a fiber knob-independent pathway for infection' J VIROL. vol. 73, no. 6, 1999, pages 5156 - 5161, XP002126062
- CRIPE T P, ET AL: 'Fiber knob modifications overcome low, heterogeneous expression of the coxsackievirus.adenovirus receptor that limits adenovirus gene transfer and oncolysis for human rhabdomyosarcoma cells' CANCER RES, vol. 61, no. 7, 2001, pages 2953 - 2960, XP002969170
- MAGNUSSON M K, ET AL: 'Genetic retargeting of adenovirus: novel strategy employing 'deknobbing' of the fiber' J. VIROL. vol. 75, no. 16, 2001, pages 7280 - 7289, XP001056142
- OKADA N: 'Efficient delivery intro denedritic cells by fiber-mutant adenovirus vectors' YAKUGAKU ZASSHI vol. 121, no. 8, 2001, pages 593 - 600, XP002976466

### SEQUENCE LISTING

<110> Fuso Pharmaceutical Industries, Ltd. et al.
<120> Novel virus vector
<130> L2228EP S3
<140> EP 04 71 1732.0
<141> February 17, 2004
<160> 13
<210> 1
   <211> 4
   <212> PRT
   <213> Artificial Sequence
<220>
<221>
<223> /note="Description of artificial sequence: fibronectin motif, peptide motif having integrin-binding activity"
<400> 1
   Arg Glu Asp Val
     1
<210> 2
   <211> 7
   <212> PRT
   <213> Artificial Sequence
<220>
   <221>
<223> /note="Description of artificial sequence: laminin motif, peptide motif having integrin-binding activity"
<400> 2
   Arg Tyr Val Val Leu Pro Arg
     1               5
<210> 3
   <211> 6
   <212> PRT
   <213> Artificial Sequence
<220>
   <221>
<223> /note="Description of artificial sequence: laminin motif, peptide motif having integrin-binding activity"
<400> 3
   Leu Gly Thr Ile Pro Gly
   1 5
<210> 4
   <211> 5
   <212> PRT
   <213> Artificial Sequence
<220>
   <221>
<223> /note="Description of artificial sequence: laminin motif, peptide motif having integrin-binding activity"
<400> 4
   Pro Asp Ser Gly Arg
   1 5
<210> 5
   <211> 5
   <212> PRT
   <213> Artificial Sequence
<220>
   <221>
<223> /note="Description of artificial sequence: laminin motif, peptide motif having integrin-binding activity"
<400> 5
   Tyr Ile Gly Ser Arg
   1 5
<210> 6
   <211> 5
   <212> PRT
   <213> Artificial Sequence
<220>
   <221>
<223> /note="Description of artificial sequence: laminin motif, peptide motif having integrin-binding activity"
<400> 6
   Ile Lys Val Ala Val
   1 5
<210> 7
   <211> 10
   <212> PRT
   <213> Artificial Sequence
<220>
   <221>
<223> /note="Description of artificial sequence: laminin motif, peptide motif having integrin-binding activity"
<400> 7
   Arg Asn Ile Ala Glu Ile Ile Lys Asp Ile
    1               5                   10
<210> 8
   <211> 4
   <212> PRT
   <213> Artificial Sequence
<220>
   <221>
<223> /note="Description of artificial sequence: motif having affinity for αVβ3 or αvβ5 integrin, peptide motif having integrin-binding activity"
<400> 4
<210> 9
   <211> 5
   <212> PRT
   <213> Artificial Sequence
<220>
   <221>
<223> /note="Description of artificial sequence: peptide motif having integrin-binding activity"
<400> 9
   Glu Ile Leu Asp Val
    1               5
<210> 10
   <211> 6
   <212> PRT
   <213> Artificial Sequence
<220>
   <221>
<223> /note="Description of artificial sequence: peptide motif having integrin-binding activity"
<400> 10
   Lys Gln Ala Gly Asp Val
    1               5
<210> 11
   <211> 4
   <212> PRT
   <213> Artificial Sequence
<220>
   <221>
<223> /note="Description of artificial sequence: peptide motif having integrin-binding activity"
<400> 11
   Asp Glu Gly Ala
    1
<210> 12
   <211> 9
   <212> PRT
   <213> Artificial Sequence
<220>
   <221> ACETYLATION
   <222> 1
   <223> Designed peptide containing peptide motif having integrin-binding activity. The N-terminal amino acid residue is acetylated. The ninth residue Xaa is bAla.
<400> 12
   Tyr Gly Gly Arg Gly Asp Thr Pro Xaa
    1               5
<210> 13
   <211> 19
   <212> PRT
   <213> Artificial Sequence
<220>
   <221> ACETYLATION
   <222> 1
   <223> Designed peptide containing peptide motif having integrin-binding activity. The N-terminal amino acid residue is acetylated.
<220>
   <221> ACETYLATION
   <222> 19
   <223> Designed peptide containing peptide motif having integrin-binding activity. The C-terminal amino acid residue is acetylated. The ninth residue Xaa1 is bAla. The eleventh residue Xaa2 is bAla.
<400> 13

## Description

### FIELD OF THE INVENTION

The present invention relates to a virus vector having a structure, in which a water-soluble polymer is linked to a surface of a virus particle and a heterogeneous peptide having an affinity for integrin is linked to the water-soluble polymer.

### BACKGROUND OF THE INVENTION

At present, an adenovirus vector, an adenovirus-associated virus vector, a retrovirus vector, a liposome and the like are known as a vector used for gene transfer. Among them, the adenovirus vector is frequently used, because it has an advantage such as 1) a high gene transfer and gene expression efficiency, 2) a possibility of gene transfer into a stationary phase cell or other many kinds of cells, 3) an availability of direct gene transfer into a tissue *in vivo*, 4) a possibility of gene transfer of a relatively large heterogeneous gene, 5) facile production of a high titer vector, and 6) a low possibility of causing cytotoxic events.

Furthermore, following steps are known as an infection mode of adenovirus: a fiber projected from a surface of a virus particle binds to an adenovirus receptor CAR (coxackie-adenovirus receptor) present on a surface of a cell to be infected, then a penton base (having five sequences of arginine(R)-glycine(G)-aspartic acid(D)) present on the surface of the virus particle binds to integrin (αVβ3, αVβ5) present on the surface of the cell and, thereby, the virus particle is endocytosed into the cell, establishing infection of adenovirus (see, T. J. Wickham et al., Cell, Vol. 73, pp.309-319, (1993)).

However, in the case that adenovirus is used as a vector for gene transfer, there are problems that 1) an inflammatory reaction is caused in an individual depending upon a dose, due to its high immunogenicity, 2) a half-life in blood is short, 3) accumulation in live is high, leading to a risk of hepatopathy, 4) a gene transfer efficiency is low for a low CAR-expressing cell (e.g., respiratory tract epithelial cells, smooth muscle cells, skeletal muscle cells, T cells, hematopoietic stem cells, dendritic cells etc.), and 5) antigenecity is high, leading to easy attack by a neutralizing antibody or a phagocyte, resulting in a low gene transfer efficiency.

Although a method of topically administrating the adenovirus vector, a genetic-engineering method of deleting an antigenic portion of adenovirus, a genetic-engineering method of modifying an adenovirus genome, and the like have been tried in order to solve above problems, not all problems were solved.

On the other hand, there have recently been reported an adenovirus vector in which polyethylene glycol (PEG) is linked to a surface of a virus particle (hereinafter, referred to as "PEG-adenovirus vector")(see, JP-A-2001-521381) for prevention of a lowered gene transfer efficiency due to actions of the neutralizing antibody and the phagocyte to a virus and for enhancement of stability in blood; an adenovirus vector in which a peptide motif having a basic sequence of arginine(R)-glycine(G)-aspartic acid(D) which is known to bind to integrin present on a surface of a target cell (hereinafter, referred to as "RGD motif') is integrated in a knob at a viral fiber tip using a genetic-engineering technique (hereinafter, referred to as "fiber-modified adenovirus vector")(see, H. Mizuguchi et al., Gene Ther., Vol. 8, pp. 730-735 (2001)); an adenovirus vector in which a peptide having a specificity for respiratory tract epithelial cells (sss. 17 peptide, SDQLASPYSHPR) is added to an outermost portion of PEG of the PEG-adenovirus vector as described above (hereinafter, referred to as "respiratory tract epithelial cell specific peptide-PEG-adenovirus vector)(see, H. Romanczuk et al., Human Gene Therapy, Vol. 10, pp. 2615-2626 (1999)) Previously, a virus vector has been described that can be derived from an adenovirus, and wherein PEG is bound to the viral surface via a linker amino acid WO 01 12235 A2. In addition, an exogenous peptide having affinity for a protein expressed on a target cell is bound to the PEG, wherein the sequence of said peptide could include an RGD motif for targeting integrin expressing cells. The linker described in WO 01 12235 A2 that associates the viral gene therapy vector and the PEG/RGD-peptide moiety is a multivalent polymer wherein said polymer consists of several glutamic acid and leucine amino acids.

However, in the PEG-adenovirus vector as described above, the binding between the virus particle and CAR is inhibited by PEG and, thereby, there arises a problem that the gene transfer efficiency in a CAR-expressing cell is lowered (see, C. R. O'riordan et al., Human Gene Therapy, Vol. 10, pp. 1349-1358 (1999)). Moreover, due to such the lowered gene transfer efficiency, there is also a possibility that the PEG-adenovirus vector administered to the tissue is not endocytosed into the target cell, and transferred by a blood stream and accumulated in liver to cause a hepatopathy.

Moreover, in the fiber-modified adenovirus vector as described above, since the RGD motif is merely inserted into the fiber of virus and, thereby, the virus vector has a similar antigenecity to that of a normal adenovirus vector, there is problem that the gene transfer efficiency is lowered due to the actions of the neutralizing antibody and the phagocyte.

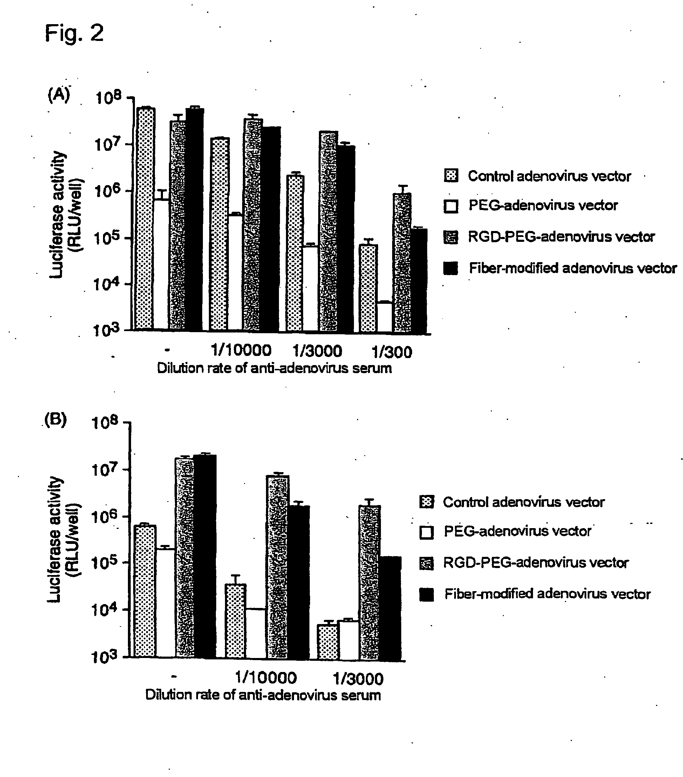

Furthermore, in the respiratory tract epithelial cell specific peptide-PEG-adenovirus vector as described above, it can transfer a gene only into the respiratory tract epithelial cell, and a substance on a surface of the respiratory tract epithelial cell which is a target of the sss.17 peptide has not been elucidated. Moreover, it is suggested that all twelve amino acid residues of the sss. 17 peptide are necessary for the binding of the vector to the respiratory tract epithelial cell in view of Table 1 and Figure 2 in H. Romanczuk *et al.,* but, generally, there is a high possibility that a peptide consisting of as long as twelve amino acid residues exhibits the immunogenicity for an administered living body. Accordingly, *in vivo* administration of the virus vector comprising the sss.17 peptide is problematic.

Furthermore, in the respiratory tract epithelial cell specific peptide-PEG-adenovirus vector as described above, there are various problems also in its method of production That is, the vector is produced by adding cysteine having an active SH group to a terminal of the sss.17 peptide to synthesize an sss.17 peptide derivative, and, separately, linking divalent hetero-reactive PEG, which has a group reactive with a lysine residue on a surface of adenovirus and a group reactive with the active SH group of the sss. 17 peptide derivative as described above respectively at each terminal of PEG, to adenovirus (PEG-adenovirus) and, thereafter, linking the sss. 17 peptide derivative as described above to the PEG-adenovirus. But, in this vector production, there were problems that the sss. 17 peptide derivatives having the active SH group are inter-molecularly cross-linked each other during the reaction and, thereby, they become impossible to be linked to PEG, that an original useful ability of adenovirus itself is lowered because the virus is exposed to a two-stage reaction system, and the like.

Therefore, development of the virus vector in which these problems or defects of the previously-used virus vectors are solved has been desired.

An object of the present invention is to provide a virus vector in which each of defects of the previously-used virus vectors is overcome while advantages thereof as described above are maintained.

In particular, an object of the present invention is to provide a virus vector in which each of defects of the PEG-adenovirus vector, the fiber-modified adenovirus vector and the respiratory tract epithelial cell specific peptide-PEG-adenovirus vector is overcome while the advantages thereof as described above are maintained. That is, an object of the present invention is to produce a virus vector while 1) an immunogenicity of the adenovirus vector is lowered to avoid an inflammatory reaction for an individual, 2) an antigenecity of the adenovirus vector is lowered to avoid an attack of a neutralizing antibody and a phagocyte, 3) a problem of a lowered gene transfer efficiency in the PEG-adenovirus vector is improved, 4) a stability of the fiber-modified adenovirus vector in blood is further enhanced, the immunogenicity of the virus vector is improved, the attack of the neutralizing antibody and the phagocyte is avoided, 5) a range of a target cell into which the respiratory tract epithelial cell specific peptide-PEG-adenovirus vector can transfer a gene is broaden, 6) production of the virus vector is made easy and efficient, 7) an advantageous ability of an original adenovirus itself is maintained, and the like.

### SUMMARY OF THE INTENTION

The inventors have intensively studied in order to solve the problems as described above and, as the result, found that a virus vector having a structure, in which a water-soluble polymer is linked to a surface of a virus particle and a heterogeneous peptide having an affinity for integrin present on a surface of a cell is linked to the water-soluble polymer, can solve the problems of a conventional virus vector for gene transfer and is useful for maintaining the advantages of the conventional virus vector, which resulted in completion of the present invention.

The present invention relates to the embodiments characterized in the claims. Thus, it relates to the following:
1. Virus vector, characterized in that a water-soluble polymer is linked to the surface of the virus particle via a linker amino acid which is cysteine and a linker having a linking ability to a thiol group and an amino acid group at one terminal, and a heterogeneous peptide containing an RGD motif and having an affinity for integrin present on a surface of a target cell is linked to the other terminal of said water-soluble polymer.
2. The virus vector according to 1, wherein said virus is adenovirus.
3. The virus vector according to 1 or 2, wherein said water-soluble polymer is polyethylene glycol or a derivative thereof.
4. The virus vector according to 3, wherein said polyethylene glycol has a molecular weight of 3000-4000.
5. The virus vector according to any one of 1 to 4, wherein said linker is *N-*(6-maleimidocaproyloxy) succinimide (EMCS).
6. The virus vector according to any one of 1 to 5, wherein said integrin is αVβ3 or αVβ5.
7. The virus vector according to any one of 1 to 6, wherein said heterogeneous peptide has a sequence containing one or more of β-alanine.
8. The virus vector according to 7, wherein said heterogeneous peptide contains lysine (K) and is branched via said lysine.
9. The virus vector according to 8, wherein said heterogeneous peptide has an amino acid sequence:
   tyrosine(Y)-glycine(G)-glycine(G)-arginine(R)-glycine(G)-aspartic acid(D)-threonine(T)-proline(P)-β-alanine(X)-lysine(K)-β-alanine(X)-proline(P)-threonine(T)-aspartic acid(D)-glycine(G)-arginine(R)-glycine(G)-glycine(G)-tyrosine(Y).
10. An in vitro method of gene transfer comprising using the virus vector as defined in any one of 1 to 9.
11. A method of production of a virus vector comprising steps of:
   a) linking a linker amino acid which is cysteine to one terminal of a water-soluble polymer to obtain a water-soluble polymer-linker amino acid;
   b) linking a heterogeneous peptide containing an RGD motif and having an affinity for integrin to the water-soluble polymer of the water-soluble polymer-linker amino acid to obtain a heterogeneous peptide-water soluble polymer-linker amino acid;
   c) linking a linker which is one having a linker ability to a thiol group and an amino group to the linker amino acid of the obtained heterogeneous peptide-water soluble polymer-linker amino acid; and
   d) linking the heterogeneous peptide-water-soluble polymer-linker amino acid with a virus via the linker.
12. The method of production of a virus vector according to 11, wherein the steps a) and b) are conducted while the linker amino acid is linked to a resin (Resin) and, thereafter, the steps c) and d) are conducted after the produced heterogeneous peptide-water soluble polymer-linker amino acid is cut from the resin.
13. The method of production of a virus vector according to 11 or 12, wherein said water-soluble polymer is polyethylene glycol or a derivative thereof.
14. A pharmaceutical composition comprising a virus vector as defined in any one of 1 to 9.
15. Use of a virus vector as defined in any one of 1 to 9 for the preparation of a pharmaceutical composition for treating cancer or adenosine deaminase deficiency via gene transfer.

According to the present invention, there is provided a vector for gene transfer, which has a low immunogenicity for an administered living body, which has a relatively long half-time in blood since it is hardly attacked by a neutralizing antibody or a phagocyte due to its low antigenecity, which can transfer a gene into a cell in a high efficiency and, thereby, which has a low accumulative property in liver, which can be produced easily and efficiently without lowering an advantageous ability of an original virus, and which is stable even after it has been repeatedly thawed and frozen after long term storage at low temperature, as well as a method of gene transfer.

A phrase used herein "a surface of a virus particle" refers to all portions of hexon and penton bases which constitute a virus coat (capsid), and a fiber and a knob projected from the penton base.

A term used herein "heterogeneous peptide" refers to a peptide which is artificially added to the virus vector.

Moreover, the term used herein "integrin" refers to a non-covalently linked transmembrane protein having a binding ability with an extracellular matrix, consisting of an α-subunit and a β-subunit. As integrin, there are known α4β1, α5β1, α8β1, αVβ1, αVβ3, αVβ6, αIIbβ3 and the like as one having an affinity for an extracellular matrix component, fibronectin, α1β1, α2β1, α3β1, α6β1, α7β1, α6β4, αVβ8 and the like as one having an affinity for laminin, α8β1, αVβ1, αVβ3, αVβ5, αVβ8, αIIbβ3 and the like as one having an affinity for vitronectin, α1β1, α2β1, αVβ8 and the like as one having an affinity for collagen, α4β1, α4β7 and the like as one having an affinity for VCAM-1, α8β1, α9β1, αVβ6 and the like as one having an affinity for tenascin-C, α4β1, αVβ3 and the like as one having an affinity for thrombospondin, αLβ2, αMβ2, αDβ2 and the like as one having an affinity for ICAM-1, αVβ3 and the like as one having an affinity for osteopontin, αVβ3, αIIbβ3, αMβ2, αXβ2 and the like as one having an affinity for fibrinogen, αEβ7 and the like as one having an affinity for E-cadherin, αVβ3, αIIbβ3 and the like as one having an affinity for a von Willebrand factor, and all of them are included in integrin of the present invention.

Furthermore, when an amino acid sequence is described in the specification, it is represented by a conventional single-letter or three-letters code.

### BRIEF DESCRIPTION OF THE DRAWINGS

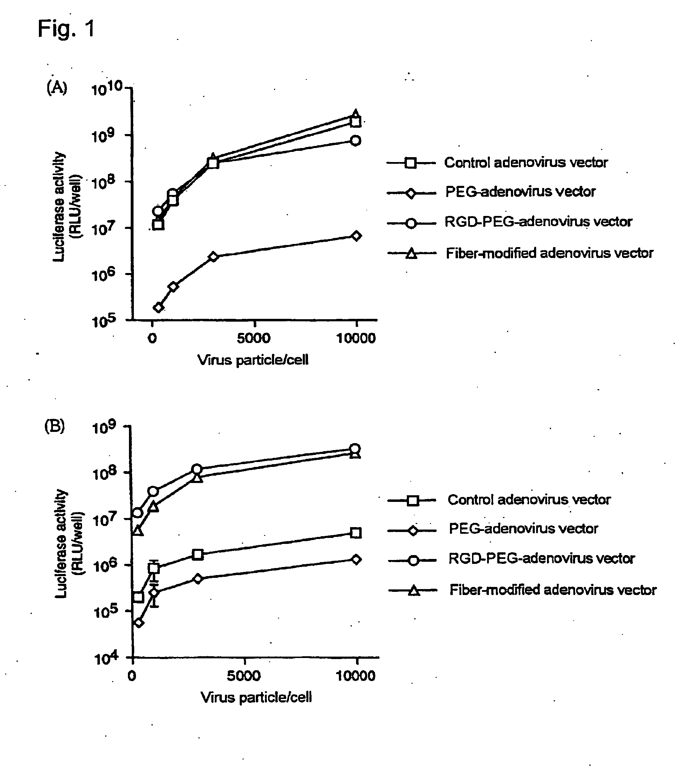

FIG 1 is a graph showing a result of a comparative experiment of a gene expression efficiency of the control adenovirus vector, the PEG-adenovirus vector, the fiber-modified adenovirus vector and the RGD-PEG-adenovirus vector, with an A549 human lung adenocarcinoma cell (ATCC: CCL-185, FIG. 1A) and a mouse melanoma B16BL6 cell (Tohoku University, Institute of Development, Aging and Cancer: TKG0598, FIG.1B).

FIG. 2 is a graph showing a result of a comparative experiment of a gene expression efficiency of the control adenovirus vector, the PEG-adenovirus vector, the fiber-modified adenovirus vector and the RGD-PEG-adenovirus vector under the presence of a neutralizing antibody, with the A549 human lung adenocarcinoma cell (FIG. 2A) and the mouse melanoma B16BL6 cell (FIG. 2B).

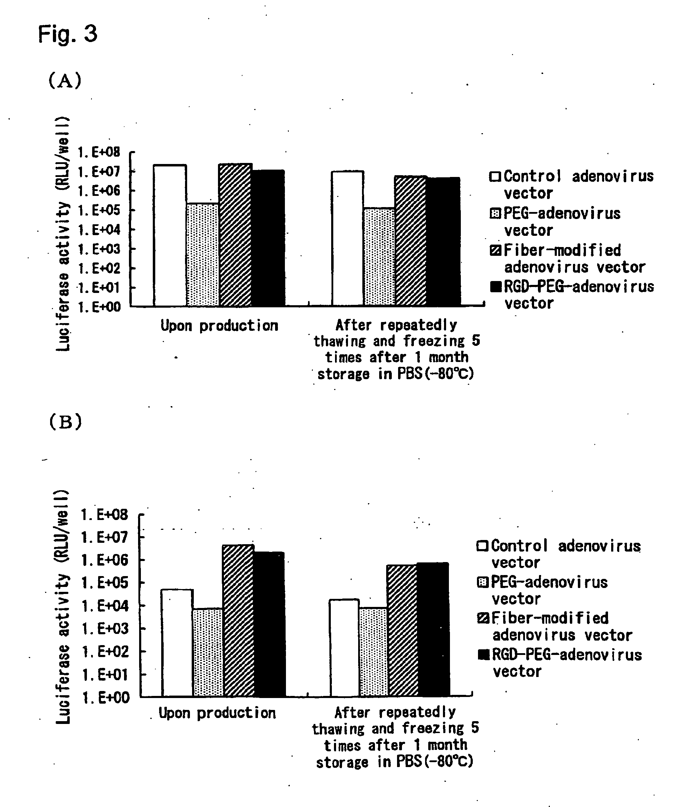

FIG. 3 is a graph showing a gene expression efficiency of the control adenovirus vector, the PEG-adenovirus vector, the fiber-modified adenovirus vector and the RGD-PEG-adenovirus vector, immediately after production, and after they have been repeatedly thawed and frozen after long term storage at low temperature, with the A549 human lung adenocarcinoma cell (FIG. 3A) and the mouse melanoma B16BL6 cell (FIG. 3B).

### DETAILED DESCRIPTION OF THE INVENTION

A virus which can be used in a virus vector of the present invention, is preferably adenovirus, retrovirus, adenovirus-associated virus or the like, and is particularly preferably adenovirus, but it is not limited thereto so long as it is known to be used in a virus vector for gene transfer. Moreover, adenovirus which is properly modified by a genetic-engineering technique may be used in a virus vector for gene transfer. For example, adenovirus in which an E1 gene region or an E3 gene region of an adenovirus genome has been deleted, adenovirus in which a heterogeneous gene has been inserted into the deleted region, and the like may be used for production of the virus vector.

A water-soluble polymer which may be used in the virus vector of the present invention, is preferably polyalkylene glycol such as polyethylene glycol, polypropylene glycol and the like having a molecular weight of 3000-4000, or a polyvinyl copolymer such as styrene-maleic acid copolymer, polyvinyl pyrrolidone, polyvinyl alcohol and the like, and is particularly preferably polyethylene glycol. Moreover, the water-soluble polymer includes a derivative in which a terminal of the water-soluble polymer is protected and/or activated. The water-soluble polymer having a protected terminal is suitable for peptide synthesis, and one protected with Fmoc (9-fluorenylmethoxycarbonyl), *t*-Boc (*tert*-butoxycarbonyl) or the like is preferably used (for example, Shearwater: Catalog Nos. 1P2Z0F02, 2Z530F02). Moreover, the water-soluble polymer having an activated terminal includes one having an active group which links with a part of an amino acid (amino, carboxyl, thio group and the like).

A heterogeneous peptide having an affinity for integrin which may be used in the virus vector as disclosed herein is preferably a peptide containing an RGD, LDV or REDV motif present in fibronectin, an RYVVLPR, LGTIPG, PDSGR, YIGSR, LRE, IKVAV, RNIAEIIKDI or RGD motif present in laminin, an RGD motif present in vitronectin, an RGD motif present in collagen, an RGD motif present in thrombin, a GPRP or RGD motif present in fibrinogen, as well as a peptide containing an EILDV, KQAGDV or DEGA motif which is reported to have an affinity for integrin.

Among the motifs having an affinity for integrin as described above, the peptide containing a fibronectin motif (RGD, LDV, REDV), a laminin motif (RYVVLPR, LGTIPG, PDSGR, YIGSR, LRE, IKVAV, RNIAEIIKDI, RGD) or a vitronectin motif (RGD) is particularly preferable.

Moreover, a peptide having an affinity for α4β1, α5β1, α8β1, αVβ1, αVβ3, αVβ6, αIIbβ3 (integrin having an affinity for fibronectin), α1β1, α2β1, α3β1, α6β1, α7β1, α6β4, αVβ8 (integrin having an affinity for laminin), α8β1, αVβ1, αVβ3, αVβ5, αVβ8, αIIbβ3 (integrin having an affinity for vitronectin) may be suitably used as the heterogeneous peptide having an affinity for integrin as disclosed herein. As the peptide, one containing a known peptide motif (for example, α5β1: RGD, α2β1: DEGA, α4β1 or α4β7: EILDV, α6β1: RGD, YIGSR or IKVAV) may be used, or it may be obtained by searching a peptide having an affinity for integrin as described above by a phage display method.

Moreover, a peptide containing a peptide having an affinity for αVβ3 or αVβ5 (for example, RGD, LDV, REDV, GPRP and the like) may be also suitably used as the heterogeneous peptide having an affinity for integrin of the present invention, since adenovirus is reported to bind to CAR and, subsequently, to αVβ3 or αVβ5.

Moreover, a peptide derivative in which an amino acid or the like is properly added to either or both of terminals of the amino acid sequence of the peptide having an affinity for integrin as described above, may be used in the present invention so long as it does not exhibit a significant antigenecity.

The virus vector as disclosed herein may be produced from the virus, the water-soluble polymer and the heterogeneous peptide as described above as an essential component, but the number of linkage, a linkage portion or linkage manner of each component between the virus particle, the water-soluble polymer and the heterogeneous peptide are not particularly limited so long as an effect of the invention is not deteriorated, and they may be properly increased, decreased or changed according to a method as described below or the known per se.

Briefly, the virus vector of the present invention may be produced by the steps of: (i) linking a linker amino acid to one terminal of a water-soluble polymer to obtain a water-soluble polymer-linker amino acid; (ii) synthesizing a heterogeneous peptide having an affinity for integrin in a stepwise manner from its carboxyl terminal, at another terminal of the water-soluble polymer of the water-soluble polymer-linker amino acid to obtain a heterogeneous peptide-water-soluble polymer-linker amino acid; (iii) linking a linker to the linker amino acid of the obtained heterogeneous peptide-water-soluble polymer-linker amino acid; and (iv) linking the heterogeneous peptide-water-soluble polymer-linker amino acid with a virus via the linker. More briefly, the water-soluble polymer may be polyethylene glycol (PEG), the linker amino acid may be cysteine, and the linker may be one having a linking ability to a thiol group and an amino group.

In a detailed description, a known peptide synthesis method (for example, a solid phase method and the like) may be used for synthesis of the heterogeneous peptide having an affinity for integrin present on a surface of the target cell. In the heterogeneous peptide of the present invention, in addition to an amino acid sequence having an affinity for integrin present on a surface of the target cell, other amino acids may be used as a spacer. In particular, one to several amino acids as a spacer, such as β-alanine, are preferably inserted between the amino acid sequence having an affinity for integrin and PEG in order to expand a distance therebetween.

More particularly, the sequence:
RGD-βAla-PEG,
RGD-βAla-βAla-PEG,
RGDTP-βAla-PEG,
YGGRGDTP-βAla-PEG,
or the like are preferable.

In the case that addition of two or more of the heterogeneous peptides to the water-soluble polymer is desired, one or more of amino acids having two amino groups may be contained in the spacer to make the heterogeneous peptide have a branched structure. Thereby, a binding ability of the virus vector of the present invention to integrin present on a surface of the target cell is enhanced. In particular, as described in the working example of the present specification, the heterogeneous peptide is preferably made to have a branched structure by inclusion of lysine between the amino acid sequence having an affinity for integrin and PEG. Moreover, more preferably, one to several amino acids, such as β-alanine, are inserted between the amino acid sequence having an affinity for integrin and lysine in order to expand the distance therebetween.

More particularly, the heterogeneous peptide preferably has a branched structure such as 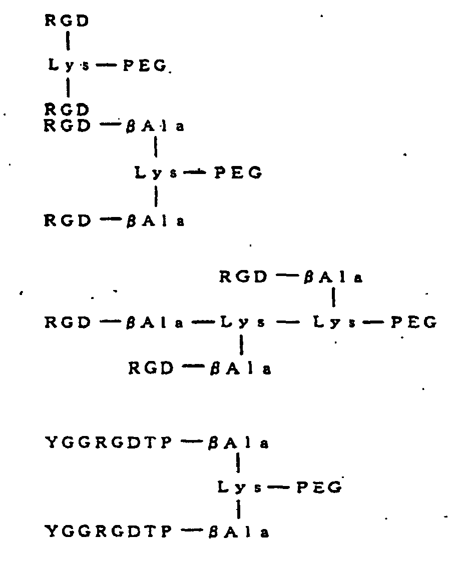 or the like.

In order to link the water-soluble polymer, to which the heterogeneous peptide having an affinity for integrin present on a surface of the target cell is linked, to a surface of the virus particle, they are preferably linked by adding at least one amino acid which is a cystein (hereinafter, referred to as "linker amino acid") which can link with a divalent linker having a linker ability to a thiol group and an amino acid group (a divalent hetero-reactive linker) to one terminal of the water-soluble polymer, to link the linker amino acid with the divalent linker and, thereafter, linking one terminal of the divalent linker with a surface of the virus particle. That is, the linker amino acid and the divalent linker are preferably placed between the water-soluble polymer and a surface of the virus particle. This method is effective in the case that the heterogeneous peptide having an affinity for integrin present on a surface of the target cell contains an acidic amino acid. This is because although activation of the terminal of the water-soluble polymer, to which the heterogeneous peptide having an affinity for integrin present on a surface of the target cell is linked, is necessary for directly linking it to a surface of the virus particle, the acidic amino acid is also activated and, thereby, an original property thereof is changed in the case that the heterogeneous peptide contains an acidic amino acid. Accordingly, in the case that the heterogeneous peptide having an affinity for integrin present on a surface of the target cell contains the acidic amino acid, the water-soluble polymer to which the heterogeneous peptide is linked is preferably linked to a surface of the virus particle with the linker amino acid and the divalent linker, without activation of the water-soluble polymer.

As the linker amino acid, an amino acid such as cysteine having a thiol group, lysine which is a basic amino acid, alanine which is a neutral amino acid, aspartic acid which is an acidic amino acid, or other amino acids may be used, but preferably it contains at least one cysteine.

An active group must be added to the terminal of the water-soluble polymer such that only the water-soluble polymer is linked to the linker amino acid. The active group to be linked with an amino group of the amino acid includes an *N*-hydroxysuccinimide group, a succinimidyl group, a carboxyl group, an aldehyde group, a benztriazole group, and the like. The active group to be linked with a carboxyl group of the amino acid includes an amino group, and the like. The active group to be linked with a thiol group of the amino acid includes a maleimide group, a vinylsulfone group, and the like. Among them, a derivative having the active group which links with the amino group of the amino acid is preferably used, and a derivative having the *N*-hydroxysuccinimide group or the succinimidyl group at the terminal thereof is particularly preferable.

If necessary, one to several amino acids may be inserted as a spacer between the linker and the water-soluble polymer. In particular, as described in the working example of the present specification, β-alanine is preferably inserted.

Next, the linker amino acid as described above, and the divalent linker which can link to the amino, carboxyl, thiol group or the like are preferably used in order to link the water soluble polymer, to which the heterogeneous peptide having an affinity for integrin present on a surface of the target cell is linked, to a surface of the virus particle. In particular, because the amino group present on a surface of the virus particle is preferable as a target for linking to a surface of the virus particle, at least, the linker having a linking ability to the amino group must be used. Moreover, in the case that cysteine is used as the linker amino acid, at least, the linker having a linking ability to the thiol group must be used.

The divalent linker includes those having a linking ability to the thiol group and the amino group (in general, it has a maleimide, *N*-hydroxysuccinimide or succinimidyl group in a molecule), for example, EMCS (*N*-(6-maleimidocaproyloxy) succinimide), GMBS (*N*-(4-maleimidobutyryloxy) succinimide), MBS (*m*-maleimidobenzyl-*N*-hydroxysuccinimide ester), SATA (*N*-succinimidyl S-acetylthioacetate), SMCC (succinimidyl 4-(*N*-maleimidomethyl)-cyclohexane-1-carboxylate), SMPB (succinimidyl 4-*p*-maleimidophenyl butyrate), SPDP (*N*-succinimidyl 3-(2-pyridylthio)propionate), Sulfo-GMBS (*N*-(γ-maleimidobutyloxy) sulfosuccinimide ester), Sulfo-LC-SPDP (sulfosuccinimidyl 6-(3'-(2-pyridyldithio) propionamide) hexanoate), Sulfo-MBS (*m*-maleimidobenzoyl-*N-*hydroxysulfosuccinimide ester), Sulfo-SMCC (sulfosuccinimidyl 4-(*N-*maleimidomethyl) cyclohexane-1-carboxylate), Sulfo-SMPB (sulfosuccinimidyl 4-(*p*-maleimidophenyl)-butyrate), and Sulfo-SBED (sulfosuccinimidyl (2-6-(biotinamido)-2-(*p*-azidobenzamido)-hexanoamido) ethyl-1,3'-dithiopropionate), which are commercially available from Techno Chemical Corporation, and the like. Moreover, *N*-succinimidyl-*N*-maleimido acetate, *N*-succinimidyl-4-(*N*-maleimido) butyrate, *N-*succimidyl-6-(*N-*maleimido) hexanoate, *N-*succinimidyl-m-(*N-*maleimido) benzoate, *N*-succinimidyl-*m*-(*N*-maleimido) benzoate, and the like may be used. Moreover, a divalent linker is disclosed herein having a linking ability to two amino groups (in general, it has an *N*-hydroxysuccinimide or succinimidyl group in a molecule), for example, BS3 (bis(sulfosuccinimidyl) suberate), DMP (dimethyl suberunudate), DMS (dimethyl suberimidate), DSG (disuccinimidyl glutarate), DSP (Loman's Reagent), DSS (disuccinimidyl suberate), DTSSP (3,3'-dithiobis(sulfosuccinimidyl propionate)), EGS (ethyleneglycol bis(succinimidyl succinate)), and Sulfo-EGS (ethylene glycol bis(succinimidyl succinate)), which are commercially available from Techno Chemical Corporation, and the like. Moreover, a divalent linker is disclosed herein having a linking ability to an amino group and a carboxyl group, for example, EDC (1-ethyl-3-(3-dimethylaminopropyl) carbodiimide), which is commercially available from Techno Chemical Corporation, and the like. Moreover, a divalent linker is disclosed herein having a linking ability to two thiol groups (in general, it has a maleimide group in a molecule), for example, BMH (bis-maleimidohexane) commercially available from Techno Chemical Corporation. Among them, the divalent linker, which is used for linking the water-soluble polymer, to which the heterogeneous peptide having an affinity for integrin present on a surface of the target cell is linked, to a surface of the virus particle, is preferably one having a linking ability to a thiol group and an amino group (in general, it has a maleimide, *N*-hydroxysuccinimide, or succinimidyl group in a molecule).

In a preferable embodiment, at first, a linker amino acid is linked to a resin (formation of a linker amino acid-resin), then the water-soluble polymer is linked to the linker amino acid (formation of a water-soluble polymer-linker amino acid-resin) and, thereafter, the heterogeneous peptide having an affinity for integrin present on a surface of the target cell is linked to the water-soluble polymer (formation of a heterogeneous peptide having an affinity for integrin -water-soluble polymer-linker amino acid-resin). Next, the heterogeneous peptide having an affinity for integrin-water-soluble polymer-linker amino acid is cut from the resin to obtain the heterogeneous peptide having an affinity for integrin-water-soluble polymer-linker amino acid. Thereafter, the divalent linker is linked to the linker amino acid (formation of the heterogeneous peptide having an affinity for integrin-water-soluble polymer-linker amino acid-divalent linker). Finally, this is linked to a surface of the virus particle (formation of a heterogeneous peptide having an affinity for integrin-water-soluble polymer-linker amino acid-divalent linker-virus particle). Such production of the virus vector of the present invention may be conducted according to the known peptide synthesis method.

In a more preferable embodiment, the linker amino acid contains cysteine, and the divalent linker has a linking ability to a thiol group and an amino group (in general, it has a maleimide, *N*-hydroxysuccinimide or succinimidyl group in a molecule). Although this method is particularly effective in the case that the heterogeneous peptide having an affinity for integrin present on a surface of the target cell contains an acidic amino acid, it is also applicable even if the heterogeneous peptide contains no acidic amino acid.

Furthermore, the heterogeneous peptide sequence to be linked to the water-soluble polymer preferably has a chain length as short as possible, in view of lowering an immunogenicity for the living body as the virus vector as described above. Moreover, one or more of the heterogeneous peptides may be contained in the virus vector in view of enhancing an affinity of the virus vector for the target cell. A chain length and the number of the heterogeneous peptide may be properly adjusted in view of the effects of the virus vector. Moreover, one or more kinds of motifs having an affinity for integrin may be contained in the heterogeneous peptide.

A measurement of a modification ratio of the water-soluble polymer in the virus vector of the present invention produced as described above may be conducted by measuring the number of amino groups remaining on the water-soluble polymer-adenovirus vector according to a fluorescamine method (A. Croyle Maria et al., Human Gene Therapy, Vol.11, pp. 1713-1722, (2000)). According to this measurement, those skilled in the art can properly determine an optimal modification ratio of the water-soluble polymer in view of the gene transfer efficiency, and can apply it to the present invention. More particularly, to a 0.42 mg/mL solution of fluorescamine in dioxane (trade name Fluram, manufactured by Fluka), a three-fold volume of 5x10¹¹ particles/mL of the water-soluble polymer-adenovirus vector is added, and the mixture is vigorously stirred. After it is incubated at room temperature for 10 minutes, fluorescence (excitation wavelength Ex: 392 nm, emission wavelength Em: 480 nm) is measured. A standard curve is produced with a non-modified adenovirus vector, and the modification ratio of the water-soluble polymer of the water-soluble polymer-adenovirus vector is calculated.

A measurement of a particle diameter of the water-soluble polymer-adenovirus vector may be conducted with ZETASIZER 3000HS (manufactured by Malvem Instruments). Similar to the modification ratio as described above, those skilled in the art can properly determine an optimal particle diameter of the virus vector in view of the gene transfer efficiency, can select the water-soluble polymer having an optimal molecular weight, and can apply it to the present invention. Moreover, the present inventors have confirmed that the modification ratio of the water-soluble polymer of the water-soluble polymer-adenovirus vector and an average particle diameter measured with ZETASIZER are increased correlatively with an amount of the water-soluble polymer added and the number of addition times. Accordingly, modification of the water-soluble polymer of the adenovirus vector can be controlled by an amount of the water-soluble polymer to be added and the number of addition times.

A measurement of the number of the virus particles may be conducted according to a method of Maizel *et al.* (J. V. Jr. Maizel et al., Virology, Vol. 36, pp. 115-125, (1968)), and those skilled in the art can properly determine the number of the viruses to be used upon gene transfer. That is, a suitable amount of a purified virus solution is collected and dissolved in 1% SDS/PBS(-), and an absorbance at OD 260 nm thereof is measured with a spectrophotomer. The number of the virus particles is calculated in terms of 1.1x10¹² particles/OD₂₆₀.

An individual, which is a subject in the case that the virus vector of the present invention is used for a therapeutic purpose, includes, for example, human, mouse, rat, hamster, guinea pig and the like. Moreover, an administration site, which is a subject of the virus vector of the present invention, includes brain, liver, kidney, spleen, prostate, small intestine, large intestine, lung, bronchial tube, skin, esophagus, stomach, duodenum, skeletal muscle and the like. Moreover, a cell, which is a subject of the virus vector of the present invention, includes cells derived from a living body (epithelial cells, muscle cells, cerebral nerve cells and the like), cancer cells, cultured cells and the like. Moreover, a cell, which is a subject in the case that the virus vector of the present invention is used *in vitro*, includes A594 cells, B16BL6 cells, HepG2 cells, COS1 cells, CHO cells and the like. Moreover, a cell, which is a subject in the case that the virus vector of the present invention is used *ex vivo*, includes T cells, B cells, hematopoietic stem cells, embryonic stem cells and the like.

Although integrin is expressed or exhibits a physiological function at various sites, at least, the following integrin appears to be expressed or to exhibit a physiological function at following sites: α1β1 at axons and lymphocytes, α2β1 at platelets and cancer cells, α3β1 at kidney, lung and cancer cells, α4β1 at lymphocytes, monocytes and acidophiles, α5β1 at various cells, α6β1 at epithelial cells, axons and cancer cells, α7β1 at skeletal muscle, α8β1 at kidney and nerve cells, α9β1 at tracheal epithelium, αVβ1 at various cells and cancer cells, αVβ3 at blood vessel and bone, αVβ5 at blood vessel and epithelium, αVβ6 at epithelium, αVβ8 at axons, α4β7 at lymphocytes, α6β4 at epithelial cells, αLβ2 at leukocytes, αMβ2 at neutrophils and monocytes, αXβ2 at monocytes and granulocytes, αDβ2 at foam cells, αIIbβ3 at platelets, αEβ7 at lymphocytes, but is not limited thereto. In general, α9β1 is expressed at tracheal epithelium, and a β2 chain is expressed on a surface of leukocytes. αLβ2 is expressed on LFA-1 (lymphocyte function-associated protein), and αMβ2 is expressed on Mac-1 (surface protein of macrophage). Moreover, a β3 chain is expressed on various cells including platelets.

The heterogeneous peptide having an affinity for integrin which is expressed site-specifically or in the cells as described above is obtained (for example, it is obtained by screening according to a phage display method or the like), and is transferred specifically into cells or sites expressing integrin as a target by applying the resultant heterogeneous peptide to the virus vector of the present invention.

In the case that the virus vector of the present invention is used *in vivo,* an administration route thereof may be property selected from topical, intravenous, mucosal, intramuscular, and oral administration to tissues or organs, or the like.

Furthermore, a therapeutic gene may be integrated in the virus vector of the present invention, such as a p53 gene (for inducing apoptosis in cancer cells), a thymidine kinase gene (for inducing apoptosis in cancer cells), an adenosine deaminase (ADA) gene (for adenosine deaminase deficiency), and the like.

In order to isolate the RGD-PEG-adenovirus vector of the present invention from a mixture of adenovirus, RGD-PEG and the RGD-PEG-adenovirus vector, for example, centrifugation with a CsCl gradient may be used. Moreover, it may be isolated by dialysis, or by a combination of ultracentrifugation with the CsCl gradient and dialysis. More particularly, a Spectrum/Pro CE (cellulose ester) Sterile Dispo Dialyzer (manufactured by SPCTRUM, molecular weight cutoff (MWCO); 300,000) may be used for isolation of the RGD-PEG-adenovirus vector. Furthermore, a validity of dialysis may be confirmed by examining whether the FITC-dextran which has a greater molecular weight than that of PEG or RGD-PEG can pass through a dialysis membrane.

### EXAMPLE

### Example 1

### Comparison of Gene Transfer Efficiency of various Adenovirus Vectors

In order to examine a gene transfer efficiency of the adenovirus vector of the present invention, various adenovirus vectors were produced and compared. That is, as illustrated in the following steps 1)-4), i) an adenovirus vector as a control, ii) a PEG-adenovirus vector, iii) a fiber-modified adenovirus vector, and iv) an adenovirus vector of the present invention were produced.

### 1) Production of Control Adenovirus Vector

A virus vector produced by Mizuguchi *et al.* was used as a control adenovirus vector. In the control adenovirus vector, E1 and E3 regions of adenovirus have been deleted, and a luciferase gene has been integrated in a deleted E1 region.

In order to propagate, isolate and purify the control adenovirus vector, at first, the virus was added together with a Dulbecco's Modified Eagle's medium (DMEM, manufactured by Sigma) with 5% fetal bovine serum to infect 293 cells. After about 2 to 3 days, the 293 cells on which CPE (cytopathic effect) was confirmed were collected together with a culture supernatant, and were centrifuged at 3000 rpm for 5 minutes. Then, obtained cells were suspended in a small amount of a culture medium, and broken by repeatedly freezing and thawing the culture four-times to liberate the virus into a solution. Thereafter, the solution was centrifuged at 3000 rpm for 5 minutes, and a supernatant was collected as a crude virus lysate (CVL). A CsCl (specific gravity 1.25/TD solution [750 mM NaCl, 50 mM KCI, 250 mM Tris, 10 mM Na₂HPO₄, pH=7.4] was poured into an SW 41 tube, and CsCl (specific gravity 1.40/TD solution) was laid under it to prepare a gradient. Then, the collected crude virus lysate was superposed on the gradient, and centrifuged at 35000 rpm for one hour at 18°C with an SW 41 rotor (manufactured by Beckman)(primary centrifugation). Thereafter, a lower white band generated in the tube was collected (primary purification). Thereafter, CsCl (specific gravity 1.34/TD solution) was poured into the SW 41 tube, the virus solution obtained in the primary purification was superposed on CsCl, and it was centrifuged with the SW 41 rotor at 35000 rpm for 18 hours at 18°C as described above (secondary centrifugation). Thereafter, the lower white band generated in the tube by the secondary centrifugation was collected (secondary purification). The virus solution obtained was placed into a dialysis tube, and the tube was placed into a phosphate buffered saline (PBS)(-) to subject to dialysis at 4°C with stirring. A dialysis solution was exchanged 3 times every 1 hour and, finally, dialysis was performed with PBS(-) with 10% glycerol for longer than 2 hours. The dialyzed virus solution was stored at -80°C until an initiation of experiment, and it was used as a control adenovirus vector. Above procedures were performed aseptically.

### 2) Production of PEG-Adenovirus Vector

For production of the PEG-adenovirus vector, the control adenovirus vector produced in the above step 1) and, thereupon, methoxy polyethylene glycol-succinimidyl propionate: (mPEG-SPA, molecular weight 5,000, manufactured by Shearwater, Catalog No.: 2M4M0D01) was used.

That is, an amount of the mPEG-SPA which corresponds to 100-fold moles relative to a primary amine present on a coat protein (hexon, penton base, fiber) of one particle adenovirus vector was added to 1x10¹² particles/ml of the control adenovirus vector, and they were reacted at 37°C for 15 minutes with stirring at 300 rpm to link the adenovirus vector to the mPEG-SPA, which was further reacted for 30 minutes under the same condition to complete the reaction. Consequently, an adenovirus vector with PEG linked thereto (PEG-adenovirus vector) was obtained.

### 3) Production of Fiber-Modified Adenovirus Vector

As a fiber-modified adenovirus vector, in which a part of an amino acid sequence present in a knob of an adenovirus fiber is modified to a peptide, arginine(R)-glycine(G)-aspartic acid(D), by a genetic-engineering method, a virus vector produced by Mizuguchi *et al*. was used (see, H. Mizuguchi et al., Gene Ther., Vol.8, pp. 730-735 (2001)). In the fiber-modified adenovirus vector, the E1 and E3 regions of the adenovirus vector have been deleted, and the luciferase gene has been integrated in the deleted E1 region. The fiber-modified adenovirus vector has a characteristic that it can also bind to a non CAR-expressing cell via integrin, and a gene can be efficiently transferred thereinto. Furthermore, propagation, isolation and purification of the fiber-modified adenovirus vector were conducted as described in the above step 1) (see, H. Mizuguchi et al., Gene Ther., Vol.8, pp.730-735 (2001)).

A vector plasmid pAdHM15 having Csp451 and Cla I restriction sites at a gene sequence portion encoding an HI loop of a fiber portion, was cut with both restriction enzymes, and then a synthetic oligo DNA corresponding to the sequence, arginine(R)-glycine(G)-aspartic acid(D), was introduced therein by *in vitro* ligation. Thereafter, the luciferase gene was inserted into the deleted E1 region. The resulting plasmid was cut with Pac I and transfected into the 293 cells to obtain a luciferase-expressing adenovirus vector having an arginine(R)-glycine(G)-aspartic acid(D) sequence in the fiber portion.

### 4) Production of Adenovirus Vector (RGD-PEG-Adenovirus Vector)

In one embodiment of the present invention, a method of production of the adenovirus vector will be illustrated with referring to a following reaction chart.

### 4-1) Synthesis of Fmoc-K(Fmoc)-PEG-βAC(Trt)-Amide Resin (compound d)

In order to synthesize (Ac-YGGRGDTPβA)₂K-PEG-βAC-amide (compound f) containing three amino acids, arginine(R)-glycine(G)-aspartic acid(D), having an affinity for integrin, at first, Fmoc-K(Fmoc)-PEG-βAC(Trt)-Amide Resin (see, Reaction Chart as described below, compound d) was synthesized. In this synthesis, cysteine (Cys) to be used as the linker amino acid is important for linking with a maleimide entity which is a divalent hetero-reactive linker specifically linking to a SH group. Moreover, β-alanine (βAla) present between the linker amino acid and PEG was used as a spacer for facilitating the reaction. Lysine (Lys) having two amino groups was introduced for linking two RGD sequences per one PEG molecule, for the purpose of enhancing an affinity of the virus vector for integrin.

The synthesis was conducted with Fmoc as a protecting group according to a solid phase method. That is, 1.5 g of Fmoc-Amide Resin (functional group content 0.66 mmol/g)(corresponding to 1.0 mmol)(manufactured by Applied Biosystems) was weighed and placed into a propylene reaction vessel (manufactured by Kokusan Chemical Co., Ltd.). The reaction vessel was set on a shaker (manufactured by IKA Co., Ltd., VIBRAX VXR), and dichloromethane (DCM) was added thereto to swell the resin. The Fmoc group was removed with 20% piperidine/DMF (*N*, *N-*dimethylformamide), and washed with DMF. A carboxylic acid of Fmoc-Cys(Trt)-OH was activated with 1 mol/L DIPC/DMF (DIPC=diisopropylcarbodiimide) and 1 mol/L HOBt/DMF (HOBt=*N*-hydroxybenzotriazole) and, thereafter, this was added to the resin to condense them (compound a). Thereafter, a condensation reaction was proceeded by repeating the procedure of removal of the Fmoc protecting group on a solid phase (deprotection), that is, freeing of an amino group→DMF washing→a reaction of a Fmoc-amino acid derivative and a freed amino group by an active ester of HOBt with a condensation reagent suitable for each step (coupling)→DMF washing. Similarly, removal of the Fmoc group (deprotection) with piperidine and condensation with Fmoc-βAla-OH (compound b) were conducted, followed by deprotection to obtain H-βAla-Cys(Trt)-Amide Resin. Then, a reaction with the Fmoc-PEG-NHS (Fmoc-PEG-NHS, manufactured by Shearwater, Catalog No. 1P2Z0F02, molecular weight 3400) was conducted with 0.45 mol/L HBTU/HOBt/DMF (HBTU=2-(1*H*-benzotriazole-1-yl)-1,1,3,3-tetramethyluronium hexafluorophosphate) as a condensation reagent under the presence of DIEA (diisopropylethylamine), since the reaction progresses very slowly only with this labile Fmoc-PEG-NHS (compound c). After deprotection with piperidine, the reaction with Fmoc-Lys(Fmoc)-OH was conducted with 1 mol/L DIPC/DMF and 1 mol/L HOBt/DMF as described above to obtain Fmoc-Lys(Fmoc)-PEG-βAla-Cys(Trt)-Amide Resin (compound d (PEG-Resin)).

### 4-2) Synthesis of [Ac-Y(Bu^{t})GGR(Pmc)GD(OBu^{t})T(Bu^{t})PβA]₂K-PEG-βAC(Trt)-Amide Resin (compound e)

Next, a peptide elongation of Fmoc-Lys(Fmoc)-PEG-βAla-Cys(Trt)-Amide Resin (compound d) produced in the step 4-1) of Example 1 was conducted by repeating deprotection and condensation on a peptide synthesizer (Model ABI 433A, synthesis program: FastMoc0.25ΩMonPrevPk) using sequentially Fmoc-βAla-OH, Fmoc-Pro-OH, Fmoc-Thr(Bu^{t})-OH, Fmoc-Asp(OBu^{t})-OH, Fmoc-Gly-OH, Fmoc-Arg(Pmc)-OH, and Fmoc-Tyr(Bu^{t})-OH. In this synthesis, βAla between RGD and PEG was used as a spacer for facilitating the reaction.

Consequently, (Fmoc-Y(Bu^{t})GGR(Pmc)GD(OBu^{t})T(Bu^{t})PβA)₂K-PEG-βAC(Trt)-Amide Resin was synthesized. Then, a free N-terminal amino group of the heterogeneous peptide containing the RGD sequence was blocked by acetylation, such that EMCS described in the following step 4-4) of Example 1 reacts only with a primary amine present on a surface of the adenovirus particle. That is, the free amino group was acetylated by the reaction with acetic anhydride under the presence of DIEA after deprotection to obtain [Ac-Y(Bu^{t})GGR(Pmc)GD(OBu^{t})T(Bu^{t})P β A]₂ K-PEG-βAC(Trt)-Amide Resin (compound e (RGD-PEG-Resin)).

### 4-3) Isolation and Purification of [Ac-Y(Bu^{t})GGR(Pmc)GD(OBu^{t})T(Bu^{t})PβA]₂K-PEG-βAC(Trt)-Amide (compound f (RGD-PEG))

A sequence [Ac-Y(Bu^{t})GGR(Pmc)GD(OBu^{t})T(Bu^{t})PβA]₂K-PEG-βAC(Trt)-Amide (compound f (RGD-PEG)) in [Ac-Y(Bu^{t})GGR(Pmc)GD(OBu^{t})T(Bu^{t})PβA]₂K-PEG-βAC(Trt)-Amide Resin (compound e (RGD-PEG-Resin)) obtained in the step 4-2) of Example 1 was isolated from Resin. That is, a crude compound f (compound f (RGD-PEG)) was obtained by cutting RGD-PEG from Resin by a treatment with a cocktail consisting of 90:5:5 of trifluoroacetic acid: TIPS (triisopropyl silane): water, and distilling off a solven, followed by freeze-drying. Then, in order to conduct purification of the crude compound f, 20 mg of this sample was dissolved in 1 mL of 10% acetonitril/ultra-purified water, the solution was centrifuged, and a supernatant was subjected to HPLC (preparative column: DAISOPAK SP-120-5-ODS-B, 20X250 mm, flow rate: 10 mL/min., mobile phase A: 0.01% trifluoroacetic acid/acetonitrile, mobile phase B: 0.01% trifluoroacetic acid/ultra-purified water, gradient: linear gradient from A/B=1/9 to A/B=7/3 over 60 minutes). Fractions at a retention time of 45 to 60 minutes were collected and combined together, and the solvent was distilled off on an evaporator. A residue was freeze-dried to obtain a pure compound f (compound f (RGD-PEG)).

### 4-4) Cross-linking of Compound f and EMCS (N-(6-maleimidocaproyloxy) succinimide)

In order to link the pure compound f (RGD-PEG) obtained in the step 4-3) of Example 1 to a surface of the virus particle, the pure compound f (RGD-PEG) was modified with EMCS, a divalent hetero-reactive reagent which can cross-link with an amino group and an SH group. Here, it is known that EMCS has a maleimide group and an *N*-hydroxysuccinimide active ester at its both terminals, and that the active ester reacts with an amino group and the maleimide group selectively reacts with an SH group.

At first, the pure compound f (RGD-PEG) was dissolved in a 10 mmol/L sodium phosphate buffer (pH 6.0), and an EMCS solution in dimethyl sulfoxide (DMSO) was added dropwise thereto. After the reaction at room temperature for 30 minutes, a 10 mmol/L sodium phosphate buffer (pH 7.4) was added to the solution, and it was frozen and stored until next use. Thus, the compound g (RDG-PEG) modified with EMCS was obtained.

### 4-5) Linking of RDG-PEG and Adenovirus Vector

The compound g (RDG-PEG) obtained in the step 4-4) of Example 1 was linked to the adenovirus vector produced in the step 1) of Example 1. That is, an amount of the compound g (RDG-PEG) which corresponds to 250-fold moles relative to the primary amine present on the coat protein (hexon, penton base, fiber) of the adenovirus vector was added to 1x10¹² particles/ml of the adenovirus vector, and they were reacted at 37°C for 15 minutes with stirring at 300 rpm to link the adenovirus vector to RDG-PEG, which was further reacted for 30 minutes under the same condition to complete the reaction. Consequently, an adenovirus vector to which the compound g is linked (RGD-PEG-adenovirus vector) was obtained.

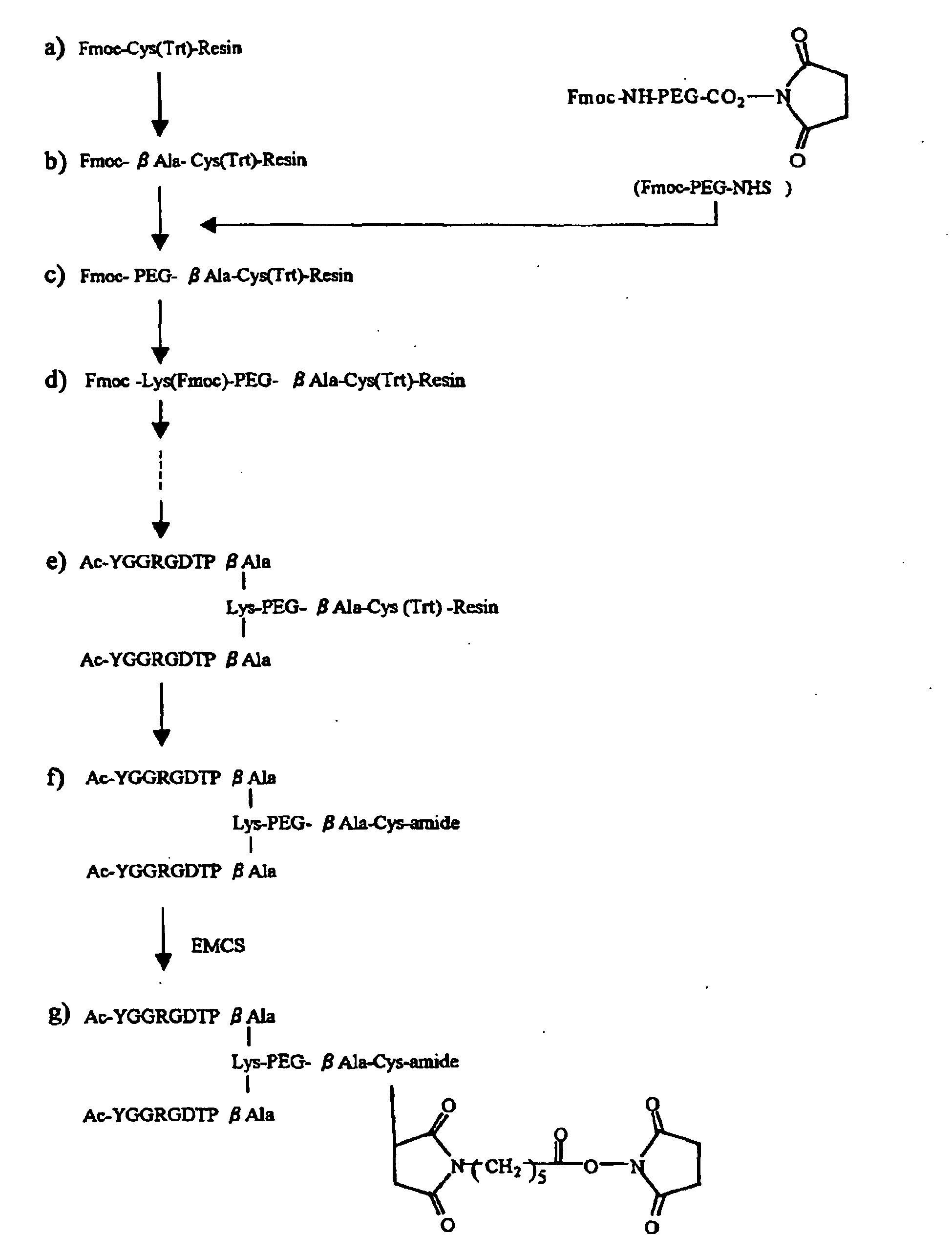

### 5) Comparative Experiment of Gene Expression Efficiency

A comparative experiment of gene expression efficiencies of the control adenovirus vector, the PEG-adenovirus vector, the fiber-modified adenovirus vector and the RGD-PEG-adenovirus vector, which were produced in the steps 1) to 4) of Example 1 as described above, was performed.

The experiment was performed with an A549 human lung adenocarcinoma cell (ATCC: CCL-185, FIG. 1A) which is a high CAR-expressing cell and a mouse melanoma B16BL6 cell (Tohoku University, Institute of Development, Aging and Cancer: TKG0598. FIG. 1B) which is a low CAR-expressing cell. The A549 cell was subcultured to subconfluent in DMEM with 10% fetal bovine serum, which was used in the experiment. Moreover, the B16BL6 cell was subcultured to subconfluent in an Eagle's minimum essential medium (MEM, manufactured by Sigma) with 7.5% fetal bovine serum, which was used in the experiment.

The A549 cell and the B16BL6 cell were seeded onto a 48-well plate at 2X10⁴ cells/0.5 mL/well and cultured for 24 hours. Each vector prepared in the medium of each cell was added to the well at 300, 1000, 3000, or 10000 particles/cell/0.5 mL, and cultured at 37°C for 24 hours under saturated vapor pressure (5% CO₂).

Moreover, a measurement of a luciferase activity as an indicator of a gene expression efficiency of the virus vector was conducted using Luciferase Assay System (manufactured by Promega) and Microlumat Plus LB96 (manufactured by Perkin Elmer) after the cells were digested in 100µL of Luciferase Cell Culture Lysis Reagent (manufactured by Promega). An activity was expressed as a Luciferase activity (RLU (relative light unit)/well).

The results thereof are shown in FIG.1 as a change in the luciferase activity when the number of the virus particles per each cell was changed. The RGD-PEG-adenovirus vector shows several hundred times higher gene expression in the A549 cell, the high CAR-expressing cell, than that of the PEG-adenovirus vector, and shows a similar degree of gene expression to that of the control adenovirus vector. Moreover, the RGD-PEG-adenovirus vector shows hundred times or more higher gene expression than that of the control adenovirus vector in the B16BL6 cell, the low CAR-expressing cell, in which a gene transfer efficiency of the control adenovirus vector is low. Furthermore, the RGD-PEG-adenovirus vector shows a similar degree of gene expression to that of the fiber-modified adenovirus vector, which has enabled the gene to be highly expressed even in the low CAR-expressing cell by inserting the RGD sequence into the fiber projected from a surface of the virus particle.

From above matters, it was found that the virus vector adsorbs onto the target cell to efficiently transfer the gene and the transferred gene can be efficiently expressed in the target cell, by adding the heterogeneous peptide having an affinity for integrin including RGD to PEG of the PEG-adenovirus vector.

It is believed that the fiber-modified adenovirus vector has approximately the same virus particle diameter and mass as those of the control adenovirus vector, since RGD has been integrated into its fiber. On the other hand, it may be said that the PEG-adenovirus vector has significantly greater virus particle diameter and mass as compared with those of the fiber-modified and control adenovirus vectors. Also, it may be said that the RGD-PEG-adenovirus vector of the present invention has further greater virus particle diameter and mass as compared with those of the PEG-adenovirus vector which contains PEG having the molecule weight of 2000, to such an extent that the RGD-PEG-adenovirus vector of the present invention contains PEG of the molecular weight of 3400 and the heterogeneous peptide having an affinity for integrin, RGD, has been integrated therein. In general, in the virus vector having a large structure like the RGD-PEG-adenovirus vector of the present invention, it is expected that a binding ability of the virus vector to integrin becomes weak even if it contains a peptide having an affinity for integrin in the structure and, thereby, expected that a gene transfer efficiency is lowered than that of the fiber-modified adenovirus vector. However, in view of the result of this working example, it is believed that the RGD-PEG-adenovirus vector having such the large structure maintains a high affinity for integrin, because it can efficiently transfer a gene as effectively as the fiber-modified adenovirus vector. It is a completely unexpected result that a similar gene transfer efficiency to that of the fiber-modified adenovirus vector is shown by addition of only three amino acids, arginine(R)-glycine(G)-aspartic acid(D), to the PEG-adenovirus vector.

From this result, it is demonstrated that even a large structured virus vector to which many water-soluble polymers have been linked can efficiently transfer a gene by linking the peptide having an affinity for integrin to the water-soluble polymer.

Moreover, since it is known that adenovirus binds to CAR present on a cell surface and, thereafter, RGD present in the penton base of the virus binds to αVβ3 and αVβ5 of the cell, it is believed that integrin, particularly αVβ3 and αVβ5 are involved also in a binding mechanism of the RGD-PEG-adenovirus vector of the present invention.

### Example 2

### Comparative Experiment of Gene Expression Efficiency of Various Adenovirus Vectors under the presence of Neutralizing Antibody

A comparative experiment of a gene transfer efficiency under the presence of a neutralizing antibody was performed for the control adenovirus vector, the PEG-adenovirus vector, the fiber-modified adenovirus vector and the RGD-PEG-adenovirus vector, which were produced in the steps 1)-4) of Example 1.

The experiment was performed with the A549 human lung adenocarcinoma cell (FIG. 2A) which is a high CAR-expressing cell and the mouse melanoma B16BL6 cell (FIG. 2B) which is a low CAR-expressing cell. The A549 cell was subcultured to subconfluent in DMEM with 10% fetal bovine serum, which was used in the experiment. Moreover, the B16BL6 cell was subcultured to subconfluent in the Eagle's minimum essential medium (MEM, manufactured by Sigma) with 7.5% fetal bovine serum, which was used in the experiment.

The A540 cell and the B16BL6 cell were seeded onto the 48-well plate at 1X10⁴ cells/0.5 mL/well and cultured for 24 hours. Each vector prepared in the medium of each cell was added to the well at 1000 particles/cell/0.5 mL under the presence or absence of ICR mouse serum and cultured at 37°C for 24 hours under the saturated vapor pressure (5% CO₂). Furthermore, a measurement of a luciferase activity was performed according to the procedure in the step 5) of Example 1. Moreover, ICR mouse serum was prepared by administering the control adenovirus vector to an ICR mouse at about 10¹⁰ particles/mouse three times.

The results thereof are shown in FIG.2 as a change in the luciferase activity when a dilution rate of anti-adenovirus serum was changed. The RGD-PEG-adenovirus maintains far higher gene expression in both of the high CAR-expressing cell and the low CAR-expressing cell under the presence of an anti-adenovirus vector antibody than that of the control adenovirus vector and the fiber-modified adenovirus vector. For example, in the A549 cell, the degrees of gene expression of the control adenovirus vector and the fiber-modified adenovirus vector are lowered to 24% and 42% under the presence of antiserum diluted to 1/10000, respectively, as a degree of their gene expression under the absence of the antibody is deemed as 100%. On the contrary, the RGD-PEG-adenovirus vector maintains 100% of gene expression.

In the B16BL6 cell, the degrees of gene expression of the control adenovirus vector, the PEG-adenovirus vector and the fiber-modified adenovirus vector are significantly lowered under the presence of antiserum diluted to 1/10000 and 1/3000. On the contrary, the RGD-PEG-adenovirus vector maintains far higher gene expression than that of other vectors. Therefore, it was elucidated that the RGD-PEG-adenovirus vector is less affected by the neutralizing antibody, and can easily transfer a gene into the target cell, as compared with the other virus vectors.

In view of above matters, it was found that the RGD-PEG-adenovirus vector of the present invention may be a superior vector for gene transfer to the fiber-modified adenovirus vector, and that the virus vector may attach to the target cell while it maintains advantages of PEG to subsequently attain efficient gene transfer and gene expression, by addition of the heterogeneous peptide having an affinity for integrin, the RGD sequence, to the virus vector via PEG.

Therefore, it was found that the PEG-adenovirus vector, to which the heterogeneous peptide having an affinity for integrin such as RGD has been added, has not only both of a high gene transfer ability and a high gene expression ability like the fiber-modified adenovirus vector, but also an antibody evading ability possessed by the PEG-adenovirus vector.

### Example 3

### Comparative Experiment of Gene Expression Efficiency of Various Adenovirus Vectors which have been repeatedly thawed and frozen after one month storage at -80°C

Each of the control adenovirus vector, the PEG-adenovirus vector, the fiber-modified adenovirus vector and the RGD-PEG-adenovirus vector, which were produced in the steps 1)-4) of Example 1, was stored in a phosphate buffered saline (PBS) at -80°C for one month. Thereafter, each of the adenovirus vectors was subjected to a step of repeatedly thawing at room temperature and freezing at -80°C five times and, thereafter, a comparative experiment of a gene expression efficiency of each virus vector was performed.

The experiment was performed with the A549 human lung adenocarcinoma cell (FIG. 3A) which is a high CAR-expressing cell, and the mouse melanoma B16BL6 cell (FIG. 3B) which is a low CAR-expressing cell. The A549 cell was subcultured to subconfluent in DMEM with 10% fetal bovine serum, which was used in the experiment. Moreover, the B16BL6 cell was subcultured to subconfluent in the Eagle's minimum essential medium (MEM, manufactured by Sigma) with 7.5% fetal bovine serum, which was used in the experiment.

The A549 cell and the B16BL6 cell were seeded onto the 48-well plate at 2X10⁴ cells/0.5 mL/well and cultured for 24 hours. Each vector prepared in the medium of each cell was added to the well at 3000 particles/cell/0.5 mL and cultured at 37°C for 24 hours under the saturated vapor pressure (5% CO₂).

Furthermore, a measurement of a luciferase activity as an indicator of a gene expression efficiency of the adenovirus vector was performed according to the procedure in the step 5) of Example 1.

The results thereof are shown in FIG. 3 as gene expression of each adenovirus vector immediately after production and after repeatedly thawing and freezing after long term storage at low temperature.

At first, it was confirmed that the PEG-adenovirus vector immediately after production has a lower gene expression efficiency in the A549 cell as compared with other virus vectors as in the step 5) of Example 1. Moreover, the control adenovirus vector, the PEG-adenovirus vector, the fiber-modified adenovirus vector and the RGD-PEG-adenovirus vector which had been repeatedly thawed and frozen five times after one month storage maintained an approximately similar degree of gene expression to that upon production.

Next, it was confirmed that the RGD-PEG-adenovirus vector immediately after production has about 100 times higher gene expression in the B16BL6 cell than that of the control adenovirus vector as in the step 5) of Example 1, and has a similar degree of gene expression to that of the fiber-modified adenovirus vector. Moreover, the RGD-PEG-adenovirus vector maintained an approximately similar degree of gene expression to that upon production, even after it had been repeatedly thawed and frozen five times after one month storage.

In view of above matters, it may be said that gene expression of the RGD-PEG-adenovirus vector of the present invention is adequately maintained even after it has been repeatedly thawed and frozen after long term storage at low temperature, and that it has a high stability.

In general, in the case that a large structure such as RGD-PEG of the present invention is added to a surface of the adenovirus vector, it is expected that a stability of the resulting vector is lowered because RGD and the adenovirus vector are linked via a linker, cysteine, PEG, lysine, and β-alanine. Nonetheless, in view of the results in the working examples, the RGD-PEG-adenovirus vector of the present invention maintained an excellent gene expression efficacy even after it had been repeatedly thawed and frozen after long term storage at low temperature. It was an unexpected result that a stability of the adenovirus vector having a large structure such as RGD-PEG is adequately maintained even after it had been repeatedly thawed and frozen after long term storage at low temperature.

According to the present invention, there is provided a vector for gene transfer, which has a low immunogenicity for a living body to be administered, which has a relatively long half-time in blood since it is less attacked by a neutralizing antibody or a phagocyte due to its low antigenecity, which can transfer a gene into a broad range of a cell in a high efficiency and, thereby, which has a low accumulative property in liver, which can be prepared easily and efficiently without lowering an advantageous ability of an original virus, and which is stable even after it has been repeatedly thawed and frozen after long term storage at low temperature, as well as a method of gene transfer.

### Sequence Listing Free Text

SEQ ID NO: 1
   Peptide motif having integrin-binding activity.
SEQ ID NO: 2
   Peptide motif having integrin-binding activity.
SEQ ID NO: 3
   Peptide motif having integrin-binding activity.
SEQ ID NO: 4
   Peptide motif having integrin-binding activity.
SEQ ID NO: 5
   Peptide motif having integrin-binding activity.
SEQ ID NO: 6
   Peptide motif having integrin-binding activity.
SEQ ID NO: 7
   Peptide motif having integrin-binding activity.
SEQ ID NO: 8
   Peptide motif having integrin-binding activity.
SEQ ID NO: 9
   Peptide motif having integrin-binding activity.
SEQ ID NO: 10
   Peptide motif having integrin-binding activity.
SEQ ID NO: 11
   Peptide motif having integrin-binding activity.
SEQ ID NO: 12
   Designed peptide containing peptide motif having integrin-binding activity.
SEQ ID NO: 13
   Designed peptide containing peptide motif having integrin-binding activity.

## Claims

1. A virus vector, **characterized in that** a water-soluble polymer is linked to the surface of the virus particle via a linker amino acid which is cysteine and a linker having a linking ability to a thiol group and an amino group at one terminal, and a heterogeneous peptide containing an RGD motif and having an affinity for integrin present on a surface of a target cell is linked to the other terminal of said water-soluble polymer.

2. The virus vector according to claim 1, wherein said virus is adenovirus.

3. The virus vector according to claim 1 or 2, wherein said water-soluble polymer is polyethylene glycol or a derivative thereof.

4. The virus vector according to claim 3, wherein said polyethylene glycol has a molecular weight of 3000-4000.

5. The virus vector according to any one of claims 1 to 4, wherein said linker is *N*-(6-maleimidocaproyloxy) succinimide (EMCS).

6. The virus vector according to any one of claims 1 to 5, wherein said integrin is αVβ3 or αVβ5.

7. The virus vector according to any one of claims 1 to 6, wherein said heterogeneous peptide has a sequence containing one or more of β-alanine.

8. The virus vector according to claim 7, wherein said heterogeneous peptide contains lysine (K) and is branched via said lysine.

9. The virus vector according to claim 8, wherein said heterogeneous peptide has an amino acid sequence:
tyrosine(Y)-glycine(G)-glycine(G)-arginine(R)-glycine(G)-aspartic acid(D)-threonine(T)-proline(P)-β-alanine(X)-lysine(K)-β-alanine(X)-proline(P)-threonine(T)-aspartic acid(D)-glycine(G)-arginine(R)-glycine(G)-glycine(G)-tyrosine(Y).

10. An in vitro method of gene transfer comprising using the virus vector as defined in any one of claims 1 to 9.

11. A method of production of a virus vector comprising steps of:
a) linking a linker amino acid which is cysteine to one terminal of a water-soluble polymer to obtain a water-soluble polymer-linker amino acid;
b) linking a heterogeneous peptide containing an RGD motif and having an affinity for integrin to the water-soluble polymer of the water-soluble polymer-linker amino acid to obtain a heterogeneous peptide-water-soluble polymer-linker amino acid;
c) linking a linker which is one having a linker ability to a thiol group and an amino group to the linker amino acid of the obtained heterogeneous peptide-water soluble polymer-linker amino acid; and
d) linking the heterogeneous peptide-water-soluble polymer-linker amino acid with a virus via the linker.

12. The method of production of a virus vector according to claim 11, wherein the steps a) and b) are conducted while the linker amino acid is linked to a resin (Resin) and, thereafter, the steps c) and d) are conducted after the produced heterogeneous peptide-water soluble polymer-linker amino acid is cut from the resin.

13. The method of production of a virus vector according to claim 11 or 12, wherein said water-soluble polymer is polyethylene glycol or a derivative thereof.

14. A pharmaceutical composition comprising a virus vector as defined in any one of claims 1 to 9.

15. Use of a virus vector as defined in any one of claims 1 to 9 for the preparation of a pharmaceutical composition for treating cancer or adenosine deaminase deficiency via gene transfer.

## Patentansprüche

1. Virusvektor, **dadurch gekennzeichnet, dass** ein wasserlösliches Polymer an einem Ende verknüpft ist mit der Oberfläche des Viruspartikels über eine Linkeraminosäure, die Cystein ist, und einen Linker, der eine Fähigkeit hat, sich mit einer Thiolgruppe und einer Aminogruppe zu verbinden, und ein heterogenes Peptid, das ein RGD-Motiv enthält und eine Affinität für Integrin hat, das auf der Oberfläche einer Zielzelle vorliegt, mit dem anderen Ende des wasserlöslichen Polymers verknüpft ist.

2. Virusvektor nach Anspruch 1, wobei das Virus ein Adenovirus ist.

3. Virusvektor nach Anspruch 1 oder 2, wobei das wasserlösliche Polymer Polyethylenglycol oder ein Derivat davon ist.

4. Virusvektor nach Anspruch 3, wobei das Polylethylenglycol ein Molekulargewicht von 3000-4000 hat.

5. Virusvektor nach einem der Ansprüche 1 bis 4, wobei der Linker *N-*(6-Maleimidocaproyloxy)-succinimid (EMCS) ist.

6. Virus vektor nach einem der Ansprüche 1 bis 5, wobei das Integrin αVβ3 oder αVβ5 ist.

7. Virusvektor nach einem der Ansprüche 1 bis 6, wobei das heterogene Peptid eine Sequenz hat, die ein oder mehrere β-Alanine enthält.

8. Virusvektor nach Anspruch 7, wobei das heterogene Peptid Lysin (K) enthält und über das Lysin verzweigt ist.

9. Virusvektor nach Anspruch 8, wobei das heterogene Peptid die Aminosäuresequenz hat:
Tyrosin(Y)-Glycin(G)-Glycin(G)-Arginin(R)-Glycin(G)-Asparaginsäure(D)-Threonin(T)-Prolin(P)-β-Alanin(X)-Lysin(K)-β-Alanin(X)-Prolin(P)-Threonin(T)-Asparaginsäure(D)-Glycin(G)-Arginin(R)-Glycin(G)-Glycin(G)-Tyrosin(Y).

10. In vitro-Verfahren eines Gentransfers, umfassend die Verwendung des Virusvektors wie in einem der Ansprüche 1 bis 9 definiert.

11. Verfahren zur Herstellung eines Virusvektors umfassend die Schritte:
a) Verknüpfen einer Linkeraminosäure, die Cystein ist, mit einem Ende eines wasserlöslichen Polymers, um eine wasserlösliches Polymer-Linkeraminosäure zu erhalten;
b) Verknüpfen eines heterogenen Peptids, das ein RGD-Motiv enthält und eine Affinität für Integrin hat, mit dem wasserlöslichen Polymer der wasserlösliches Polymer-Linkeraminosäure, um eine heterogenes Peptid-wasserlösliches Polymer-Linkeraminosäure zu erhalten;
c) Verknüpfen eines Linkers, der eine Fähigkeit hat, sich mit einer Thiolgruppe und einer Aminogruppe zu verbinden, mit der Linkeraminosäure der erhaltenen heterogenes Peptid-wasserlösliches Polymer-Linkeraminosäure; und
d) Verknüpfen der heterogenes Peptid-wasserlösliches Polymer-Linkeraminosäure mit einem Virus über den Linker.

12. Verfahren zur Herstellung eines Virusvektors nach Anspruch 11, wobei die Schritte a) und b) durchgeführt werden, während die Linkeraminosäure verbunden ist mit einem Harz (Resin), und danach die Schritte c) und d) durchgeführt werden, nachdem die hergestellte heterogenes Peptid-wasserlösliches Polymer-Linkeraminosäure von dem Harz getrennt wurde.

13. Verfahren zur Herstellung eines Virusvektors nach Anspruch 11 oder 12, wobei das wasserlösliche Polymer Polyethylenglycol oder ein Derivat davon ist.

14. Arzneimittel umfassend einen Virusvektor wie in einem der Ansprüche 1 bis 9 definiert.

15. Verwendung eines Virusvektors wie in einem der Ansprüche 1 bis 9 definiert für die Herstellung eines Arzneimittels zur Behandlung von Krebs oder Adenosindesaminase-Mangel über einen Gentransfer.

## Revendications

1. Vecteur de virus, **caractérisé en ce qu'**un polymère hydrosoluble est lié à la surface de la particule virale par un acide aminé lieur qui est une cystéine et un lieur ayant une capacité de liaison à un groupe thiol et un groupe amino à une extrémité, et un peptide hétérogène contenant un motif RGD et ayant une affinité pour l'intégrine présente sur une surface d'une cellule cible est lié à l'autre extrémité dudit polymère hydrosoluble.

2. Vecteur de virus selon la revendication 1, dans lequel ledit virus est un adénovirus.

3. Vecteur de virus selon les revendications 1 ou 2, dans lequel ledit polymère hydrosoluble est le polyéthylène glycol ou un dérivé de celui-ci.

4. Vecteur de virus selon la revendication 3, dans lequel ledit polyéthylène glycol a un poids moléculaire de 3000 à 4000.

5. Vecteur de virus selon l'une quelconque des revendications 1 à 4, dans lequel ledit lieur est le *N*-(6-maléimidocaproyloxy) succinimide (EMCS).

6. Vecteur de virus selon l'une quelconque des revendications 1 à 5, dans lequel ladite intégrine est αVβ3 ou αVβ5.

7. Vecteur de virus selon l'une quelconque des revendications 1 à 6, dans lequel ledit peptide hétérogène possède une séquence contenant une ou plusieurs β-alanines.

8. Vecteur de virus selon la revendication 7, dans lequel ledit peptide hétérogène contient une lysine (K) et est ramifié par ladite lysine.

9. Vecteur de virus selon la revendication 8, dans lequel ledit peptide hétérogène possède une séquence d'acides aminés :
tyrosine(Y)-glycine(G)-glycine(G)-arginine(R)-glycine(G)-acide aspartique (D)-thréonine(T)-proline(P)-β-alanine(X)-lysine(K)-β-alanine(X)-proline(P)-thréonine(T)-acide aspartique(D)-glycine(G)-arginine(R)-glycine(G)-glycine(G)-tyrosine(Y).

10. Procédé *in vitro* de transfert de gènes consistant à utiliser le vecteur de virus selon l'une quelconque des revendications 1 à 9.

11. Procédé de production d'un vecteur de virus comprenant les étapes consistant à:
a) lier un acide aminé lieur qui est une cystéine à une extrémité d'un polymère hydrosoluble pour obtenir un polymère hydrosoluble-acide aminé lieur ;
b) lier un peptide hétérogène contenant un motif RGD et ayant une affinité pour l'intégrine au polymère hydrosoluble du polymère hydrosoluble-acide aminé lieur pour obtenir un peptide hétérogène-polymère hydrosoluble-acide aminé lieur ;
c) lier un lieur qui est un lieur ayant une capacité de liaison à un groupe thiol et un groupe amino à l'acide aminé lieur du peptide hétérogène-polymère hydrosoluble-acide aminé lieur obtenu ; et
d) lier le peptide hétérogène-polymère hydrosoluble-acide aminé lieur avec un virus par le lieur.

12. Procédé de production d'un vecteur de virus selon la revendication 11, dans lequel les étapes a) et b) sont menées alors que l'acide aminé lieur est lié à une résine (Resin) et, ensuite, les étapes c) et d) sont menées après avoir coupé de la résine le peptide hétérogène-polymère hydrosoluble-acide aminé lieur produit.

13. Procédé de production d'un vecteur de virus selon les revendications 11 ou 12, dans lequel ledit polymère hydrosoluble est le polyéthylène glycol ou un dérivé de celui-ci.

14. Composition pharmaceutique comprenant un vecteur de virus tel que défini dans l'une quelconque des revendications 1 à 9.

15. Utilisation d'un vecteur de virus selon l'une quelconque des revendications 1 à 9 pour la préparation d'une composition pharmaceutique destinée au traitement du cancer ou d'une déficience d'adénosine désaminase par transfert de gènes.
